**Europäisches Patentamt**

**European Patent Office**

**Office européen des brevets**

(19)

(11) Veröffentlichungsnummer : **0 180 123**
**B1**

(12) # EUROPÄISCHE PATENTSCHRIFT

(45) Veröffentlichungstag der Patentschrift :
**11.01.89**

(21) Anmeldenummer : **85113311.6**

(22) Anmeldetag : **21.10.85**

(51) Int. Cl.⁴ : **C 07 D301/28**, C 07 D303/27

(54) **Verfahren zur Herstellung niedrigmolekularer Diglycidylether zweiwertiger Phenole.**

(30) Priorität : **02.11.84 DE 3439938**

(43) Veröffentlichungstag der Anmeldung :
**07.05.86 Patentblatt 86/19**

(45) Bekanntmachung des Hinweises auf die ˙Patenterteilung : **11.01.89 Patentblatt 89/02**

(84) Benannte Vertragsstaaten :
**CH DE FR GB IT LI**

(56) Entgegenhaltungen :
**DE--A-- 1 493 710**
**GB--A-- 2 006 211**
**US--A-- 3 309 384**

(73) Patentinhaber : **BAYER AG**
**Konzernverwaltung RP Patentabteilung**
**D-5090 Leverkusen 1 Bayerwerk (DE)**

(72) Erfinder : **Blazejak, Manfred, Dr.**
**Richardstrasse 81**
**D-4000 Düsseldorf (DE)**

EP 0 180 123 B1

**Beschreibung**

Die Erfindung betrifft eine Kombination aus einem einstufigen und einem zweistufigen Verfahren zur Herstellung niedrigmolekularer und niedrigviskoser Diglycidylether zweiwertiger Phenole, insbesondere des Bisphenol-A-diglycidylethers. Das Verfahren eignet sich zur Durchführung im technischen Maßstab, da es in seinem Reaktionsablauf gut beherrschbar ist und somit eine gleichmäßige Reaktionsführung und Wärmeabfuhr erlaubt und zu reproduzierbaren Ergebnissen führt.

Es ist bekannt, niedrigmolekulare und niedrigviskose Diglycidylether zweiwertiger Phenole durch Umsetzen der zweiwertigen Phenole mit überschüssigem Epichlorhydrin und festem Alkalimetallhydroxid oder anderen stark basischen Substanzen wie Calciumhydroxid oder Soda, welche vorzugsweise kontinuierlich bzw. in kleinen Portionen innerhalb von Stunden zugesetzt werden, unter vermindertem Druck (z. B. 100 bis 400 mm Hg), herzustellen. Derartige Einstufenverfahren haben sich prinzipiell bezüglich der Produktqualität bewährt. Es können so in relativ hohen Raum-Zeit-Ausbeuten Diglycidylether zweiwertiger Phenole mit hohem Gehalt an monomerem Produkt hergestellt werden (vergl. DE-AS 1 493 710). Nachteilig und bei Ansätzen in technischer Größenordnung äußerst problematisch ist jedoch das Abführen der Reaktionswärme beim Anspringen der Reaktion, unabhängig davon, ob das feste Alkalimetallhydroxid portionsweise oder kontinuierlich in das Reaktionsgemisch eingetragen wird.

Es ist weiter bekannt, mehrwertige Phenole mit überschüssigem Epichlorhydrin in Gegenwart kleiner Mengen von Katalysatoren, wie tertiären Aminen, quaternären Ammoniumverbindungen, Lithiumsalzen (FR-A-1 286 345), Thioethern oder Sulfoniumsalzen (DE-A-1 493 640) in einer ersten Stufe möglichst vollständig zu den 3-Chlor-2-hydroxypropylethern (= Chlorhydrinether) der mehrwertigen Phenole umzusetzen und diese in einer zweiten Stufe mit wäßrigem Alkalimetallhydroxid in die Polyglycidylether der mehrwertigen Phenole zu überführen. Nachteile dieser bekannten katalytischen Zweistufenverfahren können z. B. teils zu hohe Viskositäten der Endprodukte, Bildung chlorhaltiger Nebenprodukte, zu niedriger Gehalt an an Epoxidgruppen usw. sein. Das in der DE-AS 1 493 640 beanspruchte Verfahren besitzt den Nachteil relativ geringer Raum-Zeit-Ausbeuten und der für manche Anwendungen etwas zu hohen Chlorwerte der Endprodukte. Außerdem haftet den Endprodukten ein merklicher Geruch durch Schwefelverbindungen an. Erstrebenswert wären auch noch niedrigere Viskositäten, z. B. im Falle des Bisphenol-A-diglycidylethers.

Aufgabe der vorliegenden Erfindung ist nun die Bereitstellung eines Verfahrens zur Herstellung niedrigmolekularer Diglycidylether zweiwertiger Phenole, insbesondere des Bisphenol-A-diglycidylethers, das auch in technischer Größenordnung glatt und gleichmäßig abläuft und somit in seinem Reaktionsablauf gut beherrschbar ist und zu möglichst hohen Raum-Zeit-Ausbeuten und niedrigviskosem Produkt mit hohen 1,2-Epoxidgehalten und möglichst niedrigen Chlorgehalten führt.

Die Aufgabe wurde dadurch gelöst, daß man die zweiwertigen Phenole in einer ersten Reaktionsstufe mit überschüssigem Epichlorhydrin in Gegenwart einer katalytisch wirksamen Menge von Kaliumcarbonat bei vermindertem Druck und erhöhter Temperatur teilweise, das heißt zu 58-70 %, in die Bischlorhydrinether der zweiwertigen Phenole überführt, in einer zweiten Reaktionsstufe die Reaktionsmischung bei etwas tieferer Temperatur mit einer den eingesetzten zweiwertigen Phenolen im wesentlichen äquivalenten Menge an festem Natriumhydroxid behandelt und sodann die Diglycidylether der zweiwertigen Phenole in üblicher Weise isoliert.

Gegenstand der Erfindung ist somit ein Verfahren zur Herstellung eines niedrigmolekularen und niedrigviskosen Diglycidylethers eines zweiwertigen Phenols, insbesondere des Bisphenol A, durch Umsetzen des zweiwertigen Phenols mit überschüssigem Epichlorhydrin bei Unterdruck und erhöhter Temperatur in Gegenwart eines alkalischen Katalysators, Dehydrohalogenieren des als Zwischenprodukt auftretenden Bischlorhydrinethers des zweiwertigen Phenols durch kontinuierliches Eintragen einer dem eingesetzten zweiwertigen Phenol im wesentlichen äquivalenten Menge festen Natriumhydroxids in das erwärmte Reaktionsgemisch und Isolieren des Diglycidylethers des zweiwertigen Phenols, dadurch gekennzeichnet, daß man das zweiwertige Phenol mit dem Epichlorhydrin in Gegenwart von 0,5 bis 4 Gew.-% wasserfreiem Kaliumcarbonat, bezogen auf das zweiwertige Phenol, bei 100 bis 300 mbar, vorzugsweise 150 bis 250 mbar, auf 75 bis 90 °C, vorzugsweise 80 bis 85 °C, erwärmt, bis 58 bis 70 % des eingesetzten zweiwertigen Phenols umgesetzt sind, worauf man bei einer Temperatur von 60 bis 70 °C das Natriumhydroxid einträgt.

Als zweiwertige Phenole kommen beispielsweise in Frage : Resorcin ; Bis-(4-hydroxyphenyl)-methan ; 2,2-Bis-(4-hydroxyphenyl)-ethan ; 2,2-Bis-(4-hydroxyphenyl)-propan = Bisphenol A ; ω,ω'-Dibutylenglykol-bis(2-hydroxybenzoat) ; ω,ω'-Polyalkylenglykol-bis-(4-hydroxybenzoat) z. B. ω,ω'-Diethylenglykol-bis-(4-hydroxybenzoat), vorzugsweise Bisphenol A.

Pro Mol des zweiwertigen Phenols gelangen mehr als 2 Mol, vorzugsweise 6 bis 15 Mol, Epichlorhydrin zum Einsatz.

Als Katalysator wird das wasserfreie Kaliumcarbonat vorzugsweise in einer Menge von 1 bis 3 Gew.-%, bezogen auf zweiwertiges Phenol, eingesetzt.

Die Anlagerung des Epichlorhydrins an das zweiwertige Phenol zum Bischlorhydrinether des zweiwertigen Phenols in Gegenwart des Kaliumcarbonats erfolgt bei 75 bis 90 °C, vorzugsweise 80 bis 85 °C und 100 bis 300 mbar, vorzugweise 150 bis 250 mbar bis zu einem Umsatz von 58 bis 70 %, bezogen auf eingesetztes zweiwertiges Phenol.

Für das Überführen des Bischlorhydrinethers des zweiwertigen Phenols (58-70 %) und des restlichen zweiwertigen Phenols + Epichlorhydrin in den Diglycidylether des zweiwertigen Phenols wird festes Natriumhydroxid vorzugsweise in Pulver-, Plätzchen- oder Schuppenform eingesetzt, und zwar in einer dem eingesetzten zweiwertigen Phenol im wesentlichen äquivalenten Menge.

« Im wesentlich äquivalent » bedeutet hier, daß die theoretische Menge Natriumhydroxid um bis zu 4 Gew.-% überschritten werden kann. Das Eintragen des festen Natriumhydroxids erfolgt bei 60 bis 70 °C, vorzugsweise 65 bis 70 °C.

Das Aufarbeiten des Reaktionsgemisches erfolgt nach üblichen Verfahren, etwa durch Abdestillieren des überschüssig eingesetzten und nicht umgesetzten Epichlorhydrins unter verminderten Druck, wobei ca. 97 Gew.-% des unverbrauchten Epichlorhydrins wiedergewonnen werden. Der Destillationsrückstand wird zweckmässig in Toluol aufgenommen und mit Wasser verrührt.

Nach Auflösung des Kochsalzes und Abtrennen der unteren wäßrigen Phase kann das Reaktionsgut mit gesättigter Kochsalzlösung unter Zusatz von ca. 4 Gew.-% Natriumdihydrogenphosphat bis zu einem pH-Wert < 6 gewaschen werden. Es kann auch noch eine Behandlung mit einer 20 bis 30 gew.-%igen Natronlauge vorgeschaltet werden, falls der Chlorwert des Diglycidylethers über 0,4 Gew.-% liegen sollte. Nach Isolierung der mit angesäuerter Kochsalzlösung gewaschenen Toluollösung des Diglycidylethers lässt sich das Toluol abdestillieren, zuletzt bei 2-5 mbar und ca. 140 °C Sumpftemperatur.

Im Falle des Diglycidylethers des Bisphenol A betragen die Viskosität bei 25 °C ca. 7 000 bis 9 000 mPas (Höppler, Kugelfallviskosimeter), das Epoxidäquivalentgewicht ca. 183-189 und der Chlorwert ca. 0,29 bis 0,37 Gew.-%.

Das erfindungsgemäße Verfahren zur Herstellung der Diglycidylether zweiwertiger Phenole verknüpft die Vorteile der katalytischen Zweistufenverfahren, nämlich die der gleichmäßigen Reaktionsführung und stets kontrollierbaren Wärmeabführung, in hohem Maße mit den Vorteilen des Einstufenverfahrens (= Phenol/Epichlorhydrin/Alkalimetallhydroxid) bezüglich der hohen Produktqualität unter Reduzierung der Nachteile jedes der beiden Verfahren auf ein Minimum. Dieses Ergebnis war nicht vorhersehbar. Auch lag es nicht nahe, gerade wasserfreies Kaliumcarbonat als Katalysator für die Anlagerung von Epichlorhydrin an phenolische Hydroxylgruppen einzusetzen, da es, wie bei der Ausarbeitung des erfindungsgemäßen Verfahrens gefunden wurde, nur eine geringe katalytische Wirksamkeit zeigt. So ist es zum Beispiel nicht möglich, unter den Bedingungen der ersten Stufe des erfindungsgemäßen Verfahrens Bisphenol A im wesentlichen vollständig zum entsprechenden Bischlorhydrinether umzusetzen.

Durch das erfindungsgemäße Verfahren ist es möglich, Diglycidylether zweiwertiger Phenole auch im Tonnenmaßstab leicht kontrollierbar und bequem handhabbar herzustellen.

Die erfindungsgemäßen Diglycidylether können mit den bekannten Härtertypen wie cyclischen Dicarbonsäureanhydriden oder Polyaminen auf dem Elektroisoliergebiet z. B. als Vergußharze für Spulen, Kondensatoren, Isolatoren und dergleichen oder als Laminierharze und auf dem Klebstoffsektor eingesetzt werden.

Beispiel

840 kg Bisphenol A, 3 720 kg Epichlorhydrin und 20 kg wasserfreies Kaliumcarbonat werden in einem mit Stickstoff gespülten 6 000 l-Rührdruckbehälter aus VA-Stahl mit Eintragschnecke, Brüdenrohr, Kondensator und Destillatvorlage, automatischer Vakuumregelung und 6 bar Dampfheizung vorgelegt.

Unter Rühren wird der Behälterinnendruck mit Hilfe der automatischen Vakuumregelung auf etwa 200 mbar eingestellt. Daran anschließend wird die Reaktionsmischung auf 83 °C erwärmt und das Vakuum so nachgeregelt, daß das Epichlorhydrin am Rückfluß siedet.

Unter diesen Bedingungen wird die Reaktionsmischung 5 Stunden verrührt, wonach der Chlorgehalt einer vom überschüssigen Epichlorhydrin befreiten Probe 11,3 Gew.-% beträgt, was einem auf Bisphenol-A-bischlorhydrinether umgerechneten Gehalt von 65,7 Gew.-% entspricht.

Die Reaktionsmischung wird dann auf 60 °C abgekühlt und innerhalb von ca. 4 Stunden unter Stickstoff schuppenförmiges Natriumhydroxid mit der Eintragschnecke so zudosiert, daß 70 °C Sumpftemperatur nicht überschritten werden. Nach beendetem Eintragen wird 1 Stunde bei 65 bis 70 °C nachgerührt, auf 40 °C abgekühlt und überschüssig eingesetztes Epichlorhydrin im Vakuum abdestilliert, zuletzt bei 10 mbar und 100 °C Sumpftemperatur. Wenn kein Epichlorhydrin mehr überdestilliert, wird der Sumpf 1 Stunde mit Stickstoff ausgeblasen. Es werden ca. 2 945 kg Epichlorhydrin (~ 96,9 % des überschüssig eingesetzten) wiedergewonnen.

Anschließend wird das Reaktionsgut auf 80 °C abgekühlt, mit 2 000 kg Toluol und 1 800 kg Wasser versetzt, 30 Minuten bei 65 bis 75 °C verrührt und die untere wäßrige Phase nach dem Absitzen (1-2 Stunden) abgetrennt und verworfen. Die organische Phase wird mit 500 kg gesättigter Kochsalzlösung, die 20 kg Natriumdihydrogenphosphat enthält, 30 Minuten bei 65 bis 70 °C verrührt, 1 Stunde absitzen gelassen und die wäßrige Phase, die einen pH-Wert < 6 haben soll, abgetrennt. Ist dies nicht der Fall, wird der Waschvorgang wiederholt.

Anschließend wird die organische Phase andestilliert, um eine wasserfreie Lösung zu erhalten.

Es gehen etwa 1 000 l Toluol mit dem Restwasser über. Die wasserfreie Lösung wird über eine Filterpresse filtriert und anschließend vom Toluol im Vakuum bis zu einer Sumpftemperatur von 140 °C befreit.

Dann wird der Ansatz 2 Stunden bei 140 °C mit Stickstoff ausgeblasen und 4 Stunden bei 140 °C und 2 bis 5 mbar entgast. Es werden 1 205 kg (~ 96,3 d.T.) klarer, blaßgelber Diglycidylether des Bisphenol A mit folgenden Kenndaten erhalten. Viskosität $\eta$ = 8 130 mPas bei 25 °C gemessen im Kugelfallviskosimeter, Höppler ; Epoxidäquivalentgewicht : 187 ; Restchlorgehalt : 0,31 Gew.-%.

Vergleichsversuch

Wiederholt man das vorstehende Beispiel und verrührt die Reaktionsmischung aus Bisphenol A, Epichlorhydrin und Kaliumcarbonat statt 5 Stunden bei 83 °C 24 Stunden bei 83 °C, so erhält man nach dem Abdestillieren des überschüssigen Epichlorhydrins ein Reaktionsprodukt mit einem Chlorgehalt von 11,9 Gew.-%, der einem auf Bisphenol-A-bischlorhydrinether umgerechneten Gehalt von 69,2 Gew.-% entspricht. Das bedeutet, daß 19 Stunden zusätzliche Reaktionsdauer den Gehalt an Bisphenol-A-bis-chlorhydrinether lediglich um 3,5 Gew.-% erhöhen. Bei noch längerer Reaktionsdauer tritt keine weitere Steigerung der Ausbeute an Bisphenol-A-bischlorhydrinether ein.

**Patentanspruch**

Verfahren zur Herstellung eines niedrigmolekularen und niedrigviskosen Diglycidylethers eines zweiwertigen Phenols, insbesondere des Bisphenol A, durch Umsetzen des zweiwertigen Phenols mit überschüssigem Epichlorhydrin bei Unterdruck und erhöhter Temperatur in Gegenwart eines alkalischen Katalysators, Dehydrohalogenieren des als Zwischenprodukt auftretenden Bichlorhydrinethers des zweiwertigen Phenols durch kontinuierliches Eintragen einer dem eingesetzten zweiwertigen Phenol im wesentlichen äquivalenten Menge festen Natriumhydroxids in das erwärmte Reaktionsgemisch und Isolieren des Diglycidylethers des zweiwertigen Phenols, dadurch gekennzeichnet, daß man das zweiwertige Phenol mit dem Epichlorhydrin in Gegenwart von 0,5 bis 4 Gew.-% wasserfreiem Kaliumcarbonat, bezogen auf das zweiwertige Phenol, bei 100 bis 300 mbar, vorzugsweise 150 bis 250 mbar, auf 75 bis 90 °C, vorzugsweise 80 bis 85 °C, erwärmt, bis 58 bis 70 % des eingesetzten zweiwertigen Phenols umgesetzt sind, worauf man bei einer Temperatur von 60 bis 70 °C das Natriumhydroxid einträgt.

**Claim**

A process for the production of a low molecular weight, low viscosity diglycidyl ether of a dihydric phenol, more especially bisphenol A, by reaction of the dihydric phenol with excess epichlorohydrin at reduced pressure and elevated temperature in the presence of an alkaline catalyst, dehydrohalogenation of the bischlorohydrin ether of the dihydric phenol formed as intermediate product by continuous introduction of a quantity of solid sodium hydroxide substantially equivalent to the dihydric phenol used into the heated reaction mixture and isolation of the diglycidyl ether of the dihydric phenol, characterized in that the dihydric phenol is heated with the epichlorohydrin to 75 to 90 °C and preferably to 80 to 85 °C at 100 to 300 mbar and preferably at 150 to 250 mbar in the presence of 0.5 to 4 % by weight anhydrous potassium carbonate until 58 to 70 % of the dihydric phenol used have reacted, after which the sodium hydroxide used is introduced at a temperature of 60 to 70 °C.

**Revendication**

Procédé de préparation d'un éther diglycidylique à bas poids moléculaire et à basse viscosité d'un phénol divalent, en particulier du bis-phénol A, par réaction du phénol divalent avec un excès d'épichlorhydrine à température élevée en présence d'un catalyseur alcalin, déshydrohalogénation de l'éther de bis-chlorhydrine du phénol divalent obtenu en produit intermédiaire par introduction continue d'hydroxyde de sodium solide en quantité essentiellement équivalente à celle du phénol divalent mis en œuvre dans le mélange de réaction réchauffé et isolement de l'éther diglycidylique du phénol divalent, caractérisé en ce que l'on chauffe le phénol divalent avec l'épichlorhydrine en présence de 0,5 à 4 % en poids de carbonate de potassium anhydre par rapport au phénol divalent, sous une pression de 100 à 300 mbar, de préférence de 150 à 250 mbar, à des températures de 75 à 90 °C, de préférence de 80 à 85 °C, jusqu'à conversion de 58 à 70 % du phénol divalent mis en œuvre, après quoi on introduit l'hydroxyde de sodium à une température de 60 à 70 °C.